# EUROPEAN PATENT APPLICATION

(11) **EP 3 569 202 A1**
(43) Date of publication of application: **20.11.2019**
(21) Application number: 17891723.3
(22) Date of filing: 06.10.2017
(51) Int. Cl.: A61F 5/02, A61F 5/01

(54) **SACRUM-PRESSING IMPLEMENT AND SACRUM-PRESSING UNIT**

(30) Priority: 16.01.2017 JP 2017005008
(71) Applicant: Q O L Co., Ltd., Osaka-shi, Osaka 543-0052 (JP)
(72) Inventor: OKAMOTO Koichi, Osaka-shi Osaka 543-0052 (JP)
(74) Representative: Prinz & Partner mbB
(86) International application number: PCT/JP2017/036519
(87) International publication number: WO 2018/131231

(57) **Abstract**

Provided is a sacrum pressing tool that can provide improvement in transmission function between the upper body and the lower body, thus improving the motor function, through stabilization of the sacrum per se at the time of not only body rest, but also body movement or physical exercise. The sacrum pressing tool (X) includes a solid pillar-like base (21), and a pair of protruding portions (22) protruding in left-right symmetry from opposed side portions of the base (21). A width between outer side faces (22b) of the pair of protruding portions (22) is set smaller than a width between a pair of the superior posterior iliac spines (13), so that pressing faces (22a) of the pair of protruding portions (22) press a sacrum (11) solely.

## Description

### Technical Field:

This invention relates to a sacrum pressing tool and a sacrum pressing unit for pressing the sacrum of a human.

### Backgroud Art

The sacrum is located at the center of the pelvis which supports a human body between the femurs and the vertebral column. This sacrum, as joined to the vertebral column, plays an important role in stabilizing the "core" of the body. Conventionally, a sacrum pressing tool is known which is configured to correct the body core by pressing the peripheral portions of the sacroiliac joints including this sacrum, the iliac bones, etc. (see Patent Documents 1-2 for instance).

A sacrum pressing tool disclosed in Patent Document 1 includes a flat-plate-like pad having an inverted triangular shape configured to press a body area from the lumbar part to the sacrum. At the center of this pad, a reinforcing member is provided. It is described that the vertebral column is corrected by compressing the lumbar part and the sacrum while the shape of the pad is maintained by this reinforcing member.

A sacrum pressing tool disclosed in Patent Document 2 comprises a plate-like member having an abutment face for supporting the sacrum and supporting faces formed by cutting out upper opposed side portions of the abutment face for supporting the superior posterior iliac spines located on the opposed sides of the sacrum. This plate-like member will be set to a backrest board of a chair and a person will be seated on the chair with his/her sacrum being placed in abutment against the abutment face, with supporting the superior posterior iliac spines by the supporting faces. In this way, it is said that damage or injury in the lumbar vertebra can be prevented.

### Citation List

### Patent Literarture

Patent Document 1: Japanese Unexamined Patent Application Publication No. 2012-105813
Patent Document 2: Japanese Unexamined Patent Application Publication No. 2006-311946

### Summary of Invention

### Technical Problem

Between the sacrum and the iliac bones together constituting the pelvis, the sacroiliac joints are located. The contemporary populations suffer more or less developed instability of the sacroiliac joints. Consequently, the coordination between the upper body and the lower body becomes unstable, which in turn may lead to deterioration in motor function and/or onset of backache.

In the case of the sacrum pressing tool of Patent Document 1, the flat-plate-like pad is used for compressing the body area from the lumbar part to the sacrum portion, so no restraint is imposed on the movement of the sacrum. Similarly, in the case of the sacrum pressing tool of Patent Document 2, the supporting faces of the plate-like member are used for pressing the sacroiliac joints also, so no restraint is imposed on the movement of the sacrum in this case, either. This means that the conventional tools do not address to effective improvement of the motor function.

Further, with the flat-plate-like pad as used in Patent Document 1, it is not possible to press the sacrum alone in a reliable manner. On the other hand, with the sacrum pressing tool of Patent Document 2, it may be possible to prevent damage in the lumbar vertebra by pressing the sacrum at time of body rest. However, due to pressing of the sacrum and the sacroiliac joints at the same time by the supporting faces supporting the superior posterior iliac spines, the sacrum will become unstable at the time of body movement or physical exercise, thus resulting in deterioration in the transmission function between the upper body and the lower body.

In view of the above, there is a need for an improved sacrum pressing tool and unit that can provide improvement in the transmission function between the upper body and the lower body, thus improving the motor function, through stabilization of the sacrum per se at the time of not only boy rest, but also body movement or physical exercise.

### Solution to Problem

According to a characterizing feature of a sacrum pressing tool relating to the present invention, the sacrum pressing tool comprises:
a solid pillar-like base;
a pair of protruding portions protruding in left-right symmetry from opposed side portions of the base; and
a width between outer side faces of the pair of protruding portions being set smaller than a width between a pair of the superior posterior iliac spines, so that pressing faces of the pair of protruding portions press a sacrum solely.

With the above-described arrangement, a pair of protruding portions are caused to protrude in left-right symmetry from opposed side portions of a base and this base is formed like a solid pillar. Thus, with e.g. attachment of a belt to a rear face of the base for setting the protruding portions at the sacrum, it becomes possible to allow a force applied in the direction from the base toward the sacrum to be transmitted in a reliable manner to the pressing faces of the protruding portions.

In the above arrangement, a width between outer side faces of the pair of protruding portions is set smaller than a width between a pair of the superior posterior iliac spines, so that pressing faces of the pair of protruding portions press the sacrum solely. Thus, these pressing faces press only the sacrum, without pressing the sacroiliac joints present adjacent the superior posterior iliac spines, whereby the sacrum can be stabilized. Namely, as the transmission function between the upper body and the lower body is improved thanks to the stabilization of the sacrum, the pelvis too is stabilized to provide improvements in such physical functions as walking, standing, rotating (twisting), side bending, etc. Thus, the motor function is improved and onset of lumbar pain can be prevented. Further, the pressing of the sacrum alone gives one increased awareness or consciousness on the sacrum. So, a training of consciousness concentration on the so-called "*tanden*" (the inner part of the lower abdomen, just beneath the navel) is made possible also.

In this way, it has become possible to provide a sacrum pressing tool that can improve the transmission function between the upper body and the lower body and the motor function through stabilization of the sacrum per se not only at the time of body rest, but also at the time of body movement or physical exercise.

According to a further characterizing feature, the pair of protruding portions are configured such that the respective outer side faces thereof comprise inclined faces which extend closer to each other as they extend away from the base.

With the above arrangement wherein the respective outer side faces of the pair of protruding portions comprise inclined faces which extend closer to each other as they extend away from the base, these inclined faces function as "guides" for disposing the protruding portions on more inner sides that the superior posterior iliac spines, thus facilitating attachment of the sacrum pressing tool to the body. Moreover, even if the pressing faces are displaced off the sacrum at the time of physical exercise, these inclined faces will help resist tendency of the pressing faces pressing the superior posterior iliac spines inadvertently. As a result, the proper state of these pressing faces pressing the sacrum alone can be maintained more effectively.

According to a still further characterizing feature, the sacrum pressing tool further comprises a belt configured to be fixed to a rear face of the base present opposite to the protruding portions and configured also to be wound around a human body for exerting a pressing force toward the protruding portions; and
the rear face of the base is formed as a convexly curved face.

With the above arrangement, by forming the rear face of the base as a convexly curved face, when the belt is wound around a human body, this belt will come into abutment against the body along the convexly curved face of the rear face of the base, so that the pressing force applied by the pressing faces of the protruding portions to the sacrum can be effectively enhanced. Thus, the stability of the sacrum can be even further increased.

According to a still further characterizing feature, the sacrum pressing tool further comprises a belt configured to be fixed to a rear face of the base opposite to the protruding portions and configured also to be wound around a human body for exerting a pressing force toward the protruding portions; and
a pair of outer side faces located on the opposed sides of the base are formed as tapered faces that extend closer to each other as they extend away from the protruding portions.

With the above arrangement, by forming the outer side faces of the base as tapered faces that extend closer to each other as they extend away from the protruding portions, this results in reduction in the area of the rear face of the base which comes into abutment against the belt. Consequently, there is obtained a corresponding increase in the abutment force per unit area of the belt relative to the base, so that the pressing force applied by the pressing faces of the protruding portions to the sacrum can be effectively enhanced. Thus, the stability of the sacrum can be even further increased.

According to a still further characterizing feature, the pressing faces are formed as flat faces.

The sacrum extends along a convex curve toward the rear face. Then, if the pressing faces for pressing the sacrum are formed as flat faces as provided in the above arrangement, it becomes possible to concentrate the pressing force at a limited area of the convex portion. Thus, the stability of the sacrum can be even further increased.

According to a still further characterizing feature, the sacrum pressing tool further comprises a vertical extension portion that extends vertically from a top face of the base.

With the above arrangement of providing a vertical extension portion that extends vertically from a top face of the base, this vertical extension portion can be disposed along the lumbar vertebra with the pressing portions of the protruding portions being kept pressed against the sacrum alone. As a result, if a person assumes dorsal spine posture with the sacrum being kept pressed, the person will feel being compressed by the vertical extension portion, so that the person will consciously correct the dorsal spine posture, thus enhancing coordination between the lumber vertebra and the sacrum. Therefore, the transmission function between the upper body and the lower body can be improved, thus improving the motor function.

According to a characterizing feature of a sacrum pressing unit relating also to the present invention, the sacrum pressing unit comprises:
a sacrum pressing tool having a pressing face for pressing the sacrum, a width between outermost lines of the pressing face being set smaller than a width between a pair of the superior posterior iliac spines;
a lower body clothing article to which the sacrum pressing tool is fixed to an inner side of a rear portion of the article; and
a fixing position of the sacrum pressing tool relative to the lower body clothing article being set so as to allow the pressing face to press the sacrum solely.

With the sacrum pressing unit having the above-described arrangement, the unit includes a sacrum pressing tool having a pressing face for pressing the sacrum and a lower body clothing article to which the sacrum pressing tool is fixed to an inner side of a rear portion of the article; and a fixing position of the sacrum pressing tool relative to the lower body clothing article is set so as to allow the pressing face to press the sacrum solely. Thus, with only wearing of the lower body clothing article, the sacrum pressing tool will press the sacrum alone and also the lower body clothing article will prevent positional displacement of this sacrum pressing tool. Therefore, a force in the pressing face of the sacrum pressing tool toward the sacrum can be transmitted in a reliable manner. Further, since a width between outermost lines of the pressing faces is set smaller than a width between a pair of the superior posterior iliac spines, the pressing face presses only the sacrum, without pressing the sacroiliac joints present adjacent the superior posterior iliac spines, whereby the sacrum is stabilized. Namely, as the transmission function between the upper body and the lower body is improved thanks to the stabilization of the sacrum, the pelvis too is stabilized to provide improvements in such motor functions as walking, standing, twisting (rotating), side bending, etc. Thus, the motor function is improved and onset of back pain can be prevented. Further, with concentration of consciousness on the sacrum with pressing of this sacrum alone, a training of consciousness concentration on the so-called "*tanden*" (the inner part of the lower abdomen, just beneath the navel) is made possible.

In this way, it has become possible to provide a sacrum pressing unit that can improve the transmission function between the upper body and the lower body and the motor function through stabilization of the sacrum per se not only at the time of body rest, but also at the time of body movement or physical exercise.

According to a further characterizing feature, the sacrum pressing unit further comprises a fastener belt that can be wound around an outer circumferential face of the lower body clothing article; and in a rear face of the lower body clothing article at least at a position thereof where the sacrum pressing tool is to be located, there is provided a fixing portion for fixing a winding position of the fastener belt.

With the above-described arrangement, there is provided a fastener belt to be wound around an outer circumferential face of the lower body clothing article and this fastener belt is fixed to a rear face of the lower body clothing article at a position thereof where the sacrum pressing tool is to be located.

Thus, it is possible to increase the pressing force of the pressing faces of the sacrum pressing tool to the sacrum, so that the stability of the sacrum can be even further enhanced.

According to a still further characterizing feature, the sacrum pressing tool further includes a first member fixed to the rear inner side of the lower body clothing article, a second member having the pressing face and a third member detachably connected between the first member and the second member.

With the above-described arrangement, the sacrum pressing tool includes a third member which is detachably attached between a first member and a second member. This arrangement provides possibility of height adjustment. So, the pressing force of the pressing face of the sacrum pressing tool to the sacrum can be adjusted. Thus, through adjustment of the pressing force of the pressing face of the sacrum pressing tool to the sacrum, the stability of the sacrum can be enhanced in a reliable manner.

### Brief Description of Drawings

Fig. 1 is a perspective view showing a sacrum pressing tool relating to a first embodiment,
Fig. 2 is a side view showing a state when the sacrum pressing tool relating to the first embodiment is worn,
Fig. 3 is a rear view showing the state when the sacrum pressing tool relating to the first embodiment is worn,
Fig. 4 is a section taken along IV-IV in Fig. 2,
Fig. 5 is a top view of the sacrum pressing tool relating to the first embodiment,
Fig. 6 is a top view of a sacrum pressing tool relating to a second embodiment,
Fig. 7 is a top view of a sacrum pressing tool relating to a third embodiment,
Fig. 8 is a top view of a sacrum pressing tool relating to a fourth embodiment,
Fig. 9 is a top view of a sacrum pressing tool relating to a fifth embodiment,
Fig. 10 is a perspective view of a sacrum pressing tool relating to a sixth embodiment,
Fig. 11 is a perspective view of a sacrum pressing tool relating to a seventh embodiment,
Fig. 12 is a perspective view of a sacrum pressing tool relating to an eighth embodiment,
Fig. 13 is a perspective view showing showing a sacrum pressing unit,
Fig. 14 is an exploded perspective view showing a sacrum pressing tool constituting a part of a sacrum pressing unit,
Fig. 15 is a perspective view showing a sacrum pressing tool relating to a further embodiment, and
Fig. 16 is a perspective view showing the sacrum pressing tool relating to the further embodiment.

### Description of Embodiments

Next, embodiments of a sacrum pressing tool and a sacrum pressing unit both relating to the present invention will be explained with reference to the accompanying drawings. In the instant embodiments, there will be explained an example in which a belt B is attached to a rear face 21b of a sacrum pressing tool X. It should be noted however that the present invention is not limited to the following embodiments and various modifications thereof are possible within a range not departing from the essence thereof.

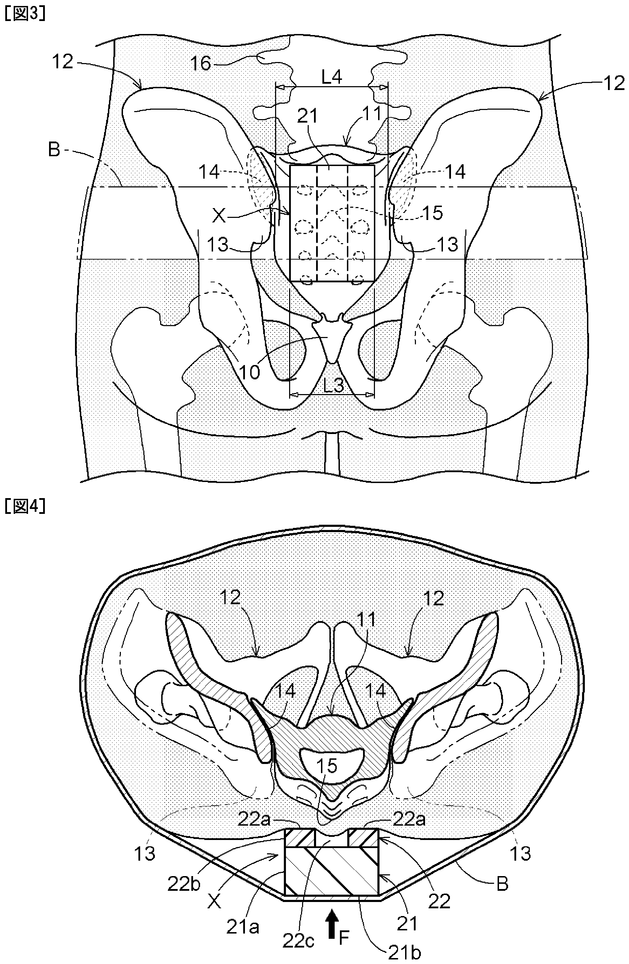

In the following explanation, in Fig. 3, a rear view showing the state when a sacrum pressing tool relating to a first embodiment is attached to the sacrum of a human body, the upper side of the plane of illustration (the direction opposite the direction of gravity) will be defined as the "upper" side and the lower side of the plane of illustration (the direction of gravity) will be defined as the "lower" side, the left side in the plane of illustration will be defined as the "left" side and the right side in the plane of illustration will be defined as the "right" side, respectively.

### [Sacrum Pressing Tool]

### [First Embodiment]

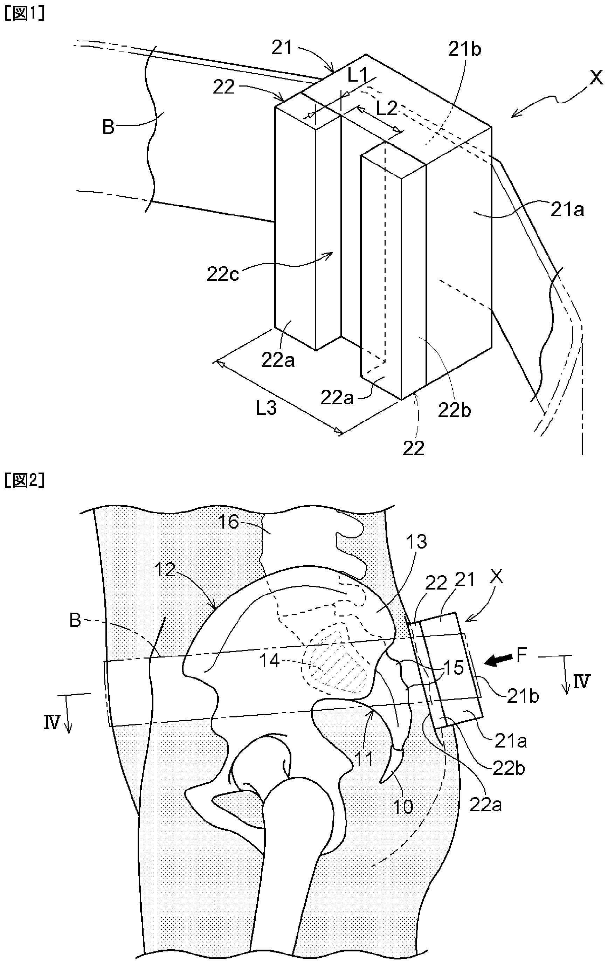

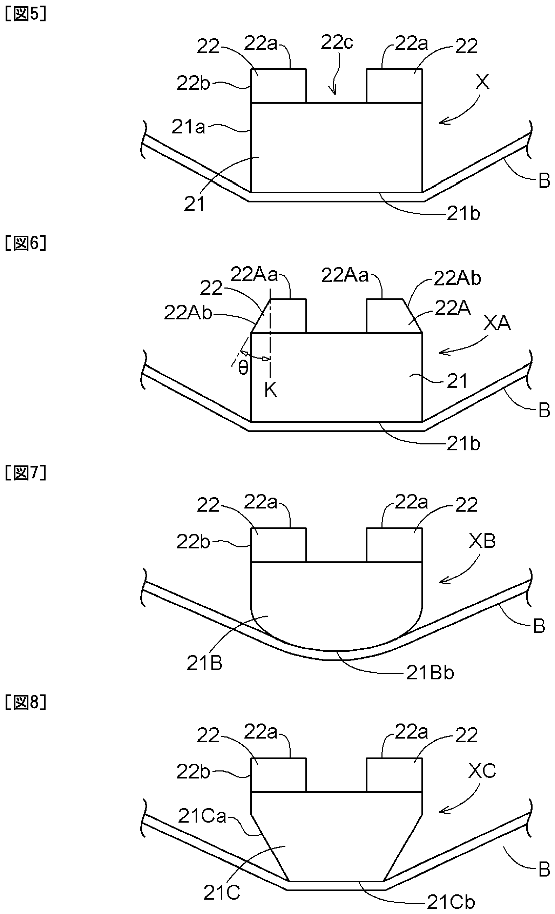

As shown in Fig. 1 and Fig. 5, the sacrum pressing tool X has a concave shape as seen from above (in the top view of Fig. 5) and includes a base 21 having a quadrangular prism-like shape and a pair of protruding portions 22 protruding in left-right symmetry from opposed sides of the base 21. Each protruding portion 22 is formed like a quadrangular prism and its front faces ("pressing faces 22a" to be described later) are formed as flat faces. To the rear face 21b of the base 21, the belt B is fixed by means of a tape, adhesive, etc. For use, the pair of protruding portions 22 of this sacrum pressing tool X will be disposed at the sacrum 11 (see Fig. 3). Under this state, the belt B will be wound around the lumbar part (waist/hip) of a user's body, whereby the rear face 21b of the base 21 will be pressed toward (against) the protruding portions 22. Further, a width L3 between respective outer side faces 22b of the pair of protruding portions 22 is set smaller than a width L4 between superior posterior iliac spines 13 of a pair of the iliac bones 12 (see Fig. 3). Incidentally, a through hole (not shown) for inserting the belt B can be formed in the base 21, so that the belt B may be inserted in this through hole of the base 21 for fixation.

Since the width L3 between the respective outer side faces 22b of the pair of protruding portions 22 is set smaller than the width L4 between the superior posterior iliac spines 13 of the pair of the iliac bones 12, when the sacrum pressing tool X is attached to a human body, the protruding portions 22 of the sacrum pressing tool X will be disposed on more inner side than the superior posterior iliac spines 13 of the iliac bones 12, so that the pressing faces 22a of the protruding portions 22 will press the sacrum 11 alone (see Figs. 2 through 4). Incidentally, the method of fixing the sacrum pressing tool X is not limited to the above-described fixing with using the belt B. Instead, the sacrum pressing tool X may be fixed by being accommodated within e.g.
spats, etc. Or, with accommodating the sacrum pressing tool X inside the spats or the like, the belt B may be wound around the rear face 21b of the sacrum pressing tool X for its fixation.

The base 21 and the protruding portions 22 as used in this embodiment are formed of thermoplastic elastomer, sponge (a foamed molded article) using synthetic rubber or synthetic resin, etc. or such material as synthetic rubber, synthetic resin, etc. and are comprised of members like solid pillars or posts that have no hollow portion therein except for pores. In the instant embodiment, the base 21 and the protruding portions 22 are formed by using different materials and the base 21 and the protruding portions 22 are bonded to each other via e.g. adhesive, etc. Also, the base 21 is provided with greater hardness than the protruding portions 22 and is formed of e.g. ethylene-vinyl acetate copolymer resin (EVA resin). Incidentally, the base 21 and the protruding portions 22 can be provided as integral unit molded of a common material (thermoplastic elastomer, etc.).

The protruding portions 22 are provided in the shape of quadrangular prisms or pillars whose height is set equal to that of the base 21. Each protruding portion 22 is disposed on more inner side than the superior posterior iliac spine of the corresponding iliac bone 12 as shown in Figs. 1-4 and includes the pressing face 22a constituted of a flat face for pressing the sacrum 11 alone and the outer side face 22b set in a same plane as the side face 21a of the base 21. Further, between the pair of protruding portions 22, there is formed a receded portion 22c and this receded portion 22c has a depth L1 and a width L2 corresponding to the shape of the median sacral ridge 15. Incidentally, this receded portion 22c can be omitted.

The width L3 between the respective outer side faces 22b (outermost lines of the pair of pressing faces 22a) of the pair of protruding portions 22 is set to 0.7 to 0.95 time, preferably, from 0.8 to 0.95 time, of the width L4 between the innermost ends of the pair of the superior posterior iliac spines 13 (see Fig. 3). If the width L3 between the outer side faces 22b of the pair of pressing faces 22a is set to 0.95 time or less of the width L4 between the innermost ends of the pair of superior posterior iliac spines 13, even if there occurs slight positional displacement of the pressing face 22a relative to the sacrum 11 in the left/right direction, the pressing face 22a will not press the superior posterior iliac spine 13. Further, if the width L3 between the outer side faces 22b of the pair of pressing faces 22a is set to 0.7 time or more of the width L4 between the innermost ends of the pair of superior posterior iliac spines 13, the pressing area of the pressing face 22a to the sacrum 11 can be maintained appropriate, whereby the pressing posture is stabilized. Moreover, if the width L3 between the outer side faces 22b of the pair of protruding portions 22 were set too small, this would result in increase in the skin contact pressure of the pressing face 22a, thus presenting risk of inviting blood circulation problem. Incidentally, the width L4 between the innermost ends of the pair of superior posterior iliac spines 13 differs from one individual to another. So, the width L3 between the outer side faces 22b of the pair of protruding portions 22 will be set after actual determination of the width L4 in advance.

Incidentally, the protruding portion 22, as long as it has the pressing face 22a located on more inner side than the superior posterior iliac spine 13 and pressing the sacrum 11 alone, can have a vertical height smaller than that of the base 21, and the outer side face 22b of the protruding portion 22 can be retracted to more inner side than the side face 21a of the base 21.

In this way, as the pair of protruding portions 22 are caused to protrude in left-right symmetry from the opposed sides of the base 21 and this base 21 is formed like a solid pillar, when the belt B is attached to the rear face 21b of the base 21 to dispose the protruding portions 22 at the sacrum 11, by the fastening force of the belt B, the force transmitted from the base 21 toward the sacrum 11 in a direction F can be transmitted effectively to the pressing faces 22a of the protruding portions 22 (see Fig. 2 and Fig. 4). Moreover, in the instant embodiment, since the base 21 is provided with greater hardness than the protruding portions 22, the above force in the direction F from the base 21 toward the sacrum 11 will be hardly reduced by the base 21, but will be transmitted in a reliable manner to the pressing faces 22a of the protruding portions 22.

In the instant embodiment, the protruding portion 22 is disposed on more inner side than the superior posterior iliac spine 13 and this protruding portion 22 has the pressing face 22a which presses only the sacrum 11. This arrangement has a function of preventing hindrance of functioning of the sacroiliac joint 14. Namely, by pressing the sacrum 11 alone, it is possible to cope with both the problem of excessive mobility (excessive mobility) of the sacroiliac joint 14 and the problem of retrograde degeneration (lack of mobility) thereof, so that improvement/enhancement of the load transmission function can be expected. As an example, in case rapid left and right alternations of functioning of the sacroiliac joint 14 are required such as in the case of a walking, a running, etc., the focused pressing of the sacrum 11 alone provides stability in the supporting leg and helps the appropriate load transmitting function, so that smooth motion of lower limbs and appropriate load transmission at the lumbosacral joints are made possible. Consequently, improvement in the motor function and prevention, improvement of backache, etc. can be expected.

The sacroiliac joint 14 in general is a flat-face joint and also a synovial membrane joint. This means that it is subject to massive amount of neural control. In fact, in the sacroiliac joint 14, a large number of proprioceptors are present in distribution throughout. Thus, by the function of the pressing faces 22a of the protruding portions 22 configured to press the sacrum 11 solely, it becomes possible to apply a stimulation to these proprioceptors. With this, specifically, a "nodding" motion, a "rise-up" motion, of the sacrum 11 and sacrum stability will be induced instinctively. Distinct from the objects of the conventional wearing tool which reside in fixation, correction and support, the principal object of the present invention is to induce motion improvement through improvement/enhancement of load transmission function. Namely, the invention provides improvement/enhancement of motor function not only at the time of body rest, but also at the time of body movement or physical exercise.

The conventional "hip belt" or "pelvis belt" were configured to apply a pressure such as increase of stomach pressure, so that load may be reduced through limitation of the mobility of the lumber vertebra or pelvis. For this reason, deterioration in the load transmission function would result in exchange for the reduction of load to a particular portion obtained by excessive restraint on the mobility of the lumbar part (waist/hip). Whereas, the sacrum pressing tool X according to the present embodiment is substantially free from such mobility restraint, so it can be used for an extended period, including the time of physical exercise.

Next, other embodiments will be explained with reference to the accompanying drawings. The basic arrangements or configurations of these further embodiments are identical to those of the foregoing embodiment. So, only differences thereof will be explained with reference to the drawings. Incidentally, for the sake of readiness of understanding, the following explanation will be given with using the same names and reference numerals/marks as those used in the foregoing embodiment.

### [Second Embodiment]

Fig. 6 is a top view which shows a sacrum pressing tool XA relating to a second embodiment. In this embodiment, respective outer side faces 22Ab of a pair of protruding portions 22A are formed as inclined faces which extend closer to each other as they extend away from the base 21. More particularly, the outer side faces 22Ab are formed by cutting away pressing face 22Aa side outer corner portions of the protruding portions 22 in the form of triangular prisms. Further, preferably, an angle θ formed between a virtual plane K perpendicular to the pressing face 22Aa of the protruding portion 22A and the outer side face 22Ab is provided as an acute angle of 45 degrees or less.

With the above arrangement, the amount of bulging of the outer side face 22Ab toward the superior posterior iliac spine 13 can be suppressed, so that it becomes possible to prevent pressing of the superior posterior iliac spine 13 by the protruding portion 22A while securing the pressing area of the pressing face 22Aa. Incidentally, as shown in Fig. 6, the inclined face of the outer side face 22Ab of the protruding portion 22A is provided as a flat face. However, there is no particular limitation in this respect, and it can be a curved face instead.

In this embodiment, the inclined face of the outer side face 22Ab functions as a "guide" for disposing the protruding portion 22A on inner side of the superior posterior iliac spine 13, so that attachment of the sacrum pressing tool XA to the body is facilitated. Moreover, even if the pressing faces 22Aa are displaced off the sacrum 11 at the time of physical exercise, these inclined faces will effectively resist the tendency of the pressing faces 22Aa pressing of the superior posterior iliac spines 13. As a result, the proper state of the pressing faces 22Aa pressing the sacrum 11 alone can be maintained more effectively. The rest of the configuration, functions and effects are same as those of the first embodiment, so detailed explanation thereof will be omitted.

### [Third Embodiment]

Fig. 7 is a top view which shows a sacrum pressing tool XB relating to a third embodiment. In this embodiment, a rear face 21Bb of a base 21B is formed as a convexly curved face. Incidentally, in this embodiment too, like the second embodiment, the respective outer side faces 22b of the pair of protruding portions 22 may be formed as inclined faces which extend closer to each other as they extend away from the base 21B.

In this embodiment, since the belt B will come into abutment along the convexly curved face of the rear face 21Bb of the base 21B, the pressing force applied by the pressing faces 22a of the protruding portions 22 to the sacrum 11 can be effectively enhanced. Thus, the stability of the sacrum 11 can be even further increased. Consequently, the motor function can be even enhanced. The rest of the configuration, functions and effects are same as those of the first embodiment, so detailed explanation thereof will be omitted.

### [Fourth Embodiment]

Fig. 8 is a top view which shows a sacrum pressing tool XC relating to a fourth embodiment. In this embodiment, a pair of outer side faces 21 Ca of a base 21C are formed as tapered faces that extend closer to each other as they extend away from the protruding portions 22. In other words, the outer side faces 21Ca are formed with cutting away opposed corner portions of these outer side faces 22Ca and a rear face 21Cb of the base 21 in the form of triangular prisms. Incidentally, in this embodiment too, like the second embodiment, the respective outer side faces 22b of the pair of protruding portions 22 may be formed as inclined faces which extend closer to each other as they extend away from the base 21C.

In this embodiment, the area of contact of the belt B with the rear face 21Cb of the base 21C is smaller than that in the first embodiment. Consequently, there is obtained a corresponding increase in the abutment force per unit area of the belt B to the base 21C, so that the pressing force applied by the pressing faces 22a to the sacrum 11 can be effectively enhanced. Thus, the stability of the sacrum 11 can be even further increased. Consequently, the motor function can be even enhanced. The rest of the configuration, functions and effects are same as those of the first embodiment, so detailed explanation thereof will be omitted.

### [Fifth Embodiment]

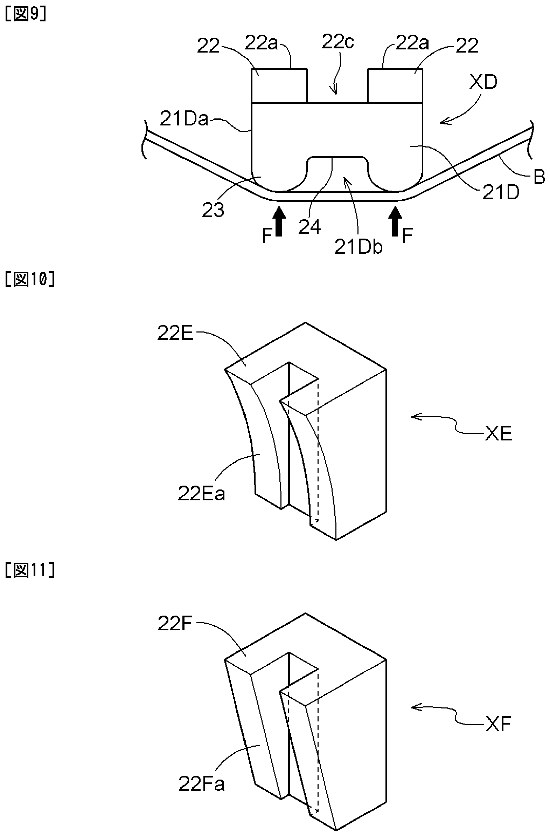

Fig. 9 is a top view which shows a sacrum pressing tool XD relating to a fifth embodiment. In this embodiment, a rear face 21Db of a base 21D is receded and this rear face 21Db includes a pair of curved face portions 23 extending continuously from respective side faces 21Da and a groove portion 24 formed between the curved face portions 23. Incidentally, the curved face portions 23 can be provided in the form of quadrangular prisms. And, the shape of the groove portion 24 is not particularly limited. Further, in this embodiment too, like the second embodiment, the respective outer side faces 22b of the pair of protruding portions 22 may be formed as inclined faces which extend closer to each other as they extend away from the base 21D.

In this embodiment, since the pair of curved face portions 23 and the pair of protruding portions 22 are disposed in correspondence with each other so as to follow the direction F from the base 21 toward the sacrum 11. Thus, the abutment force of the belt B to the base 21D can be transmitted in an efficient manner to the pressing faces 22a of the protruding portions 22. Thus, the stability of the sacrum 11 can be even further increased. Consequently, the motor function can be even enhanced. The rest of the configuration, functions and effects are same as those of the first embodiment, so detailed explanation thereof will be omitted.

### [Sixth Embodiment]

Fig. 10 is a perspective view which shows a sacrum pressing tool XE relating to a sixth embodiment. In this embodiment, pressing faces 22Ea of protruding portions 22E are formed as curved faces extending along the rear face contour of the lumbar part from the coccyx 10 to the sacrum 11. With this arrangement, the sacrum pressing tool XE can be worn with a natural comfortable feel. The rest of the configuration, functions and effects are same as those of the first embodiment, so detailed explanation thereof will be omitted. Incidentally, the arrangements of the foregoing second through fifth embodiments can be used in any appropriate combinations as well.

### [Seventh Embodiment]

Fig. 11 is a perspective view which shows a sacrum pressing tool XF relating to a seventh embodiment. In this embodiment, pressing faces 22Fa of protruding portions 22F are formed as flat inclined faces such that the thickness of the protruding portion 22F progressively decreases. With this arrangement, even when some individual difference exists in the curved contour of the sacrum 11, it is still possible to cause the pressing faces 22Fa to generally follow the rear face contour of the lumbar part from the coccyx 10 to the sacrum 11. Thus, degree of freedom in wearing the sacrum pressing tool XF without discomfort can be increased. The rest of the configuration, functions and effects are same as those of the first embodiment, so detailed explanation thereof will be omitted. Incidentally, the arrangements of the foregoing second through fifth embodiments can be used in any appropriate combinations as well.

### [Eighth Embodiment]

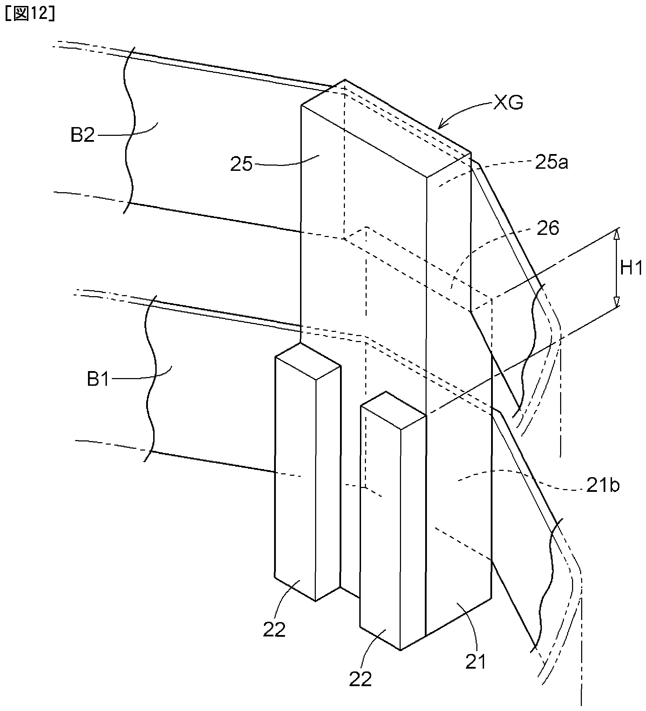

Fig. 12 is a perspective view which shows a sacrum pressing tool XG relating to an eighth embodiment. In this embodiment, there is additionally provided a vertical extension portion 25 that extends vertically from the top face of the base 21 used in the first embodiment. Further, as a rear face 25a of this vertical extension portion 25 is receded from the rear face 21b of the base 21 toward the protruding portion 22, a stepped portion 26 is formed. A height H1 of this stepped portion 26 from the top faces of the protruding portions 22 is determined in accordance with a body shape of each individual so as to prevent displacement of the sacrum pressing tool XG. To the rear face 21b of the base 21, a first belt B1 (similar to the belt B described above) is fixed with a tape, adhesive, or the like. To the rear face 25a of the vertical extension portion 25, a second belt B2 is fixed with a tape, adhesive, or the like. The lower end of this second belt B2 is fixed as being placed in abutment against the upper face of the stepped portion 26. Incidentally, in the vertical extension portion 25, a through hole (not shown) may be formed to allow insertion therethrough of the second belt B2 into the vertical extension portion 25 to be fixed therein. Further, a belt worn on pants or the like may be used as the second belt B2, also.

When the sacrum pressing tool XG is to be put into use, the first belt B1 will be wound and worn around the user's lumbar part, whereby the rear face 21b of the base 21 will be pressed toward the protruding portions 22. If a physical exercise is effected under this condition, due to the downwardly curved contour of the sacrum 11, the sacrum pressing tool XG can be displaced upwardly. Then, in the instant embodiment, such displacement of the sacrum pressing tool XG is prevented by winding the second belt B2 also around the user's body. So that, the proper state of the protruding portions 22 pressing the sacrum 11 alone can be maintained in a reliable manner. In particular, since the lower end of the second belt B2 is placed in abutment against the top face of the stepped portion 26, the displacement of the sacrum pressing tool XG can be prevented in a reliable manner.

Moreover, when the vertical extension portion 25 is disposed along the lumber vertebra 16, the posture of this lumber vertebra 16 can be corrected. As a result, if the user assumes dorsal spine posture with the sacrum 11 being kept pressed, he/she will feel being compressed by the vertical extension portion 25, so that he/she will consciously correct the dorsal spine posture, thus enhancing coordination between the lumber vertebra 16 and the sacrum 11. The rest of the configuration, functions and effects are same as those of the first embodiment, so detailed explanation thereof will be omitted. Incidentally, the arrangements of the foregoing second through seventh embodiments can be used in any appropriate combinations as well.

### [Other Embodiments]

In the foregoing embodiments, the base 21 and the protruding portions 22 are provided in the form of quadrangular prisms. Instead, they may be provided in the form of other pillar-like bodies with corner portions thereof being cut away in arcuate shape or in the form of cylinders or oval posts as well.

### [Sacrum Pressing Unit]

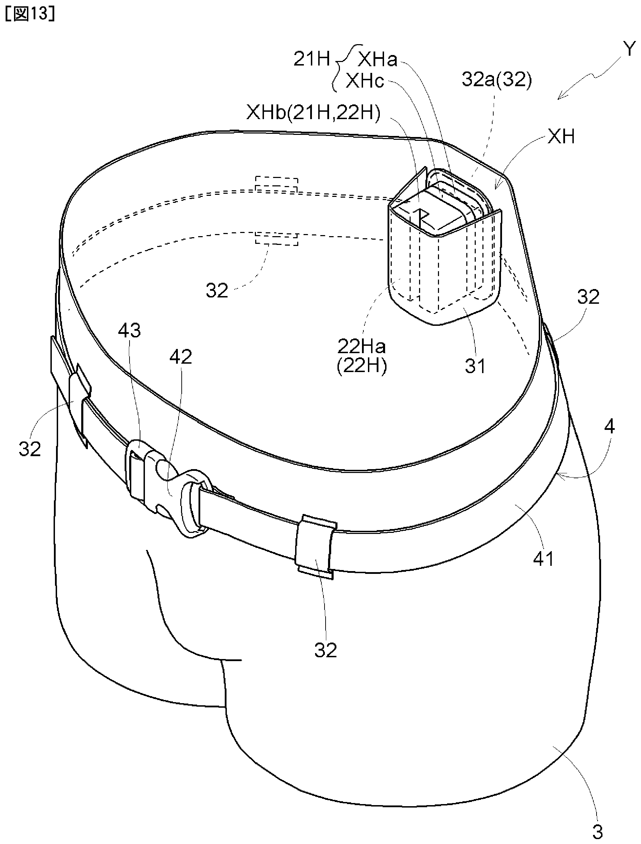

As shown in Fig. 13, a sacrum pressing unit Y relating to this embodiment includes a sacrum pressing tool XH including a solid pillar-like base 21H and a pair of protruding portions 22H protruding in left-right symmetry and having pressing faces 22H at the leading ends thereof and formed as as flat faces for pressing the sacrum 11 alone, spats 3 (an example of "lower body clothing article") having, on an inner side of a rear portion thereof, a pocket 31 for accommodating the sacrum pressing tool XH, and a fastener belt 4 that can be wound around an outer circumferential face of the spats 3. Incidentally, the lower body clothing article to which the sacrum pressing tool XH is to be fixed is not limited to the spats 3, but can be any other underwear, pants, a skirt, etc. also.

The arrangement of the protruding portions 22H of the sacrum pressing tool XH is same as the protruding portions 22 used in the foregoing first embodiment, except that the protruding portions 22H comprise pillar-like bodies having outer end corner portions thereof being cut away in arcuate shape. Thus, detailed explanation thereof will be omitted herein. Incidentally, the shape of the protruding portions 22H can also be same or similar as/to that of the second embodiment shown in Fig. 6, that of the sixth embodiment shown in Fig. 10, or that of the seventh embodiment shown in Fig. 11.

The sacrum pressing tool XH employed in the instant embodiment includes a first member XHa fixed to the pocket 31 and constituting a part of the base 21H, a second member XHb constituting another part of the base 21H and a pair of protruding portions 22H, and a third member XHc detachably connected between the first member XHa and the second member XHb and constituting a part of the base 21H. These members, i.e. the first member XHa, the second member XHb, and the third member XHc, are formed respectively integrally of elastic members made of flexible material such as thermoplastic elastomer, etc.

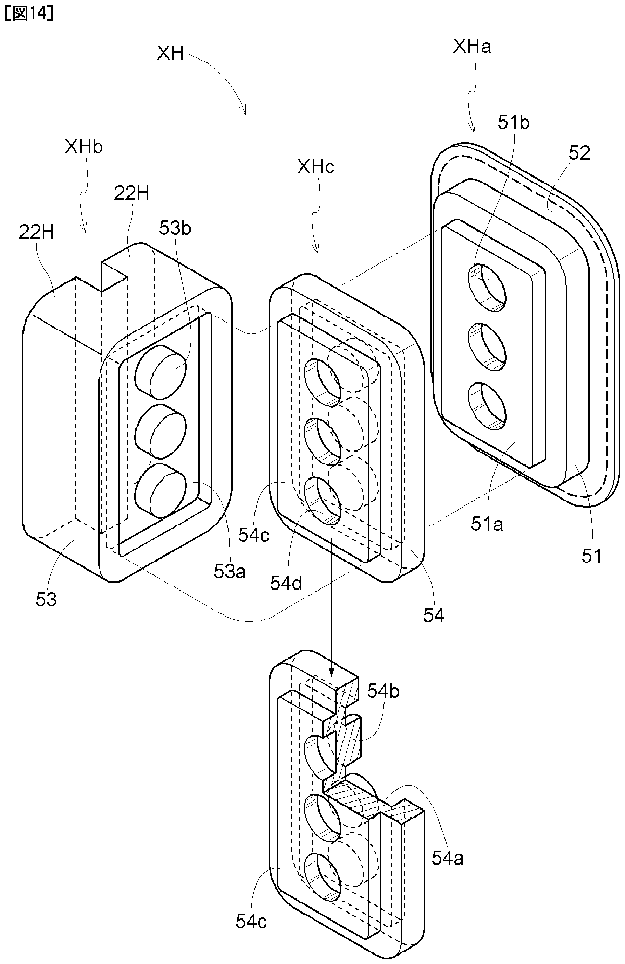

As shown in Fig. 14, the first member XHa includes a first main body portion 51 having a quadrangular prism-like shape, and an annular sewn portion 52 extending in the form of a flat plate from one end of the first main body portion 51 to the outer side in a circumferential direction and fixed by sewing, etc. to the inner side of the rear portion of the spats 3. Further, on the other end of the first main body portion 51, there is formed a first protruding end portion 51a having a protruding surface. And, in the surface of this first protruding end portion 51a, a plurality of (three in this embodiment) first receded portions 51b receded in the cylindrical form are provided.

The second member HXb includes a second main body portion 53 which constitutes a part of the base 21H and the pair of protruding portions 22H extending integrally in left-right symmetry from opposed sides of one end of the second main body portion 53. At the other end of the second main body portion 53, there is formed a first rectangular receded portion 53a formed as a rectangular-shaped recess in the surface, and on the bottom of this first rectangular receded portion 53a, a plurality of (three in this embodiment) cylindrical first protruding portions 53b are formed.

The third member XHc includes a third main body portion 54 in the form of a quadrangular prism. At one end of the third main body portion 54, there is formed a second rectangular receded portion 54a which is receded in the rectangular shape in the surface. And, on the bottom of this second rectangular receded portion 54a, a plurality of (three in this embodiment) cylindrical second protruding portions 54b are formed. At the other end of the third main body portion 54, there is formed a second protruding end portion 54c whose surface is protruded. And, on the surface of this second protruding end portion 54c, there are provided a plurality (three in this embodiment) of second receded portions 54d in the form of cylindrical recesses. Incidentally, the shape of the rear face of the third member XHc can be same as that of the fourth embodiment shown in Fig. 8 or that of the fifth embodiment shown in Fig. 9.

With the above-described arrangements, the first protruding end portion 51a of the first member XHa and the second rectangular receded portion 54a of the third member XHc will be engaged with each other, with the first receded portions 51b and the second rectangular receded portions 54b being fitted to each other. Also, the second protruding end portion 54c of the third member XHc and the second rectangular receded portion 54a of the second member XHb will be engaged with each other,
with the second receded portions 54d and the first protruding portions 53b being fitted to each other. On the other hand, in case the third member XHc is not needed, the first protruding end portion 51a of the first member XHa and the second rectangular receded portion 54a of th second member XHb will be engaged with each other, with the first receded portions 51b and the first protruding portions 53b being fitted to each other. In this way, with this detachable provision of the third member XHc between the first member XHa and the second member XHb, it becomes possible to adjust the height of the sacrum pressing tool XH, so that the pressing force of the pressing faces 22Ha of the protruding portions 22H to the sacrum 11 can be adjusted. Incidentally, the manner of connection between the first member XHa or the second member XHb and the third member XHc is not particularly limited. For instance, with omission of the receded portions 51b, 54d and the protruding portions 53b, 54b, the connection may comprise solely engagement between the protruding end portion 51a, 54c and the rectangular receded portion 53a, 54a or may be any other type of engagement as well.

As shown in Fig. 13, the spats 3 are formed of a material having flexibility and elasticity such as polyurethane, etc. So that, when a person wears the spats 3, the pocket 31 accommodating the sacrum pressing tool XH will be located at the position of the sacrum 11. This pocket 31 is provided for preventing dropping of the sacrum pressing tool XH. However, this pocket 11 can be omitted.

Further, in the outer circumferential face of the spats 3, a plurality (five in this embodiment) of fixing portions 32 are sewn to receive insertion of a winding portion 41 of the fastener belt 4 to be described later for fixing winding positions of this fastener belt 4. These fixing portions 32 are formed of a material having non-elastic resin or cloth fabric and has a hole sized equal to the width of the winding portion 41 of the fastener belt 4. The fixing portions 32 used in this embodiment are set such that the fastener belt 4 may be located slightly downwardly of the superior posterior iliac spines so as to enhance attachment wearability of the fastener belt 4. With this, no vertical displacement of the fastener belt 4 fixed via the fixing portions 32 will occur. So that, the pressing force of the pressing faces 22Ha of the protruding portions 22H to the sacrum 11 can always be maintained constant. In particular, in the instant embodiment, a fixing portion 32a is provided in the rear face at the position where the pocket 31 of the spats 3 is located. With this, the pressing force from the pressing faces 22Ha of the protruding portions 22H to the sacrum 11 can be increased for further enhancing the stability of the sacrum 11.

The fastener belt 4 is formed of a material such as a resin, a cloth fabric, having no elasticity and includes the winding portion 41 to be wound around the outer circumferential face of the spats 3, a first fastening member 42 fixed to one end of the winding portion 41 and a second fastening member 43 provided at the other end of the winding portion 41 and capable of adjusting the length of the winding portion 41. When the fastener belt 4 is to be worn, the winding portion 41, under a loosened state thereof, will be retained temporarily to the second fastening member 43. Then, after connecting the first fastening member 42 to the second fastening member 43, the other end of the winding portion 41 will be pulled tense, whereby the sacrum pressing tool XH accommodated in the spats 3 will be moved toward the sacrum 11, so that the pressing force of the pressing faces 22Ha of the protruding portions 22H to the sacrum 11 can be increased.

In this way, with the sacrum pressing unit Y according to the instant embodiment, only with wearing the spats 3, the sacrum pressing tool XH will press the sacrum 11 alone and as this sacrum pressing tool XH is fixed to the spats 3, positional displacement of the sacrum pressing tool XH can be prevented. As a result, thanks to the elastic contracting force inherent in the spats 3, the force effective from the base 21H toward the sacrum 11 can be transmitted in a reliable manner to the pressing faces 22Ha of the protruding portions 22H.

Based on the above-described embodiments, following arrangements can be contemplated.
(1) A sacrum pressing unit Y comprising the sacrum pressing tool XH including the solid pillar-like base 21H and the pair of protruding portions 22H protruding in left-right symmetry from the opposed sides of the base 21H, the spats 3 with the sacrum pressing tool XH being fixed to the inner side of its rear portion, the width between the respective outer side faces of the protruding portions 22H being set smaller than the width between the pair of superior posterior iliac spines 13, the fixing position of the sacrum pressing tool XH to the spats 3 being set such that the pressing faces 22Ha of the pair of protruding portions 22H press only the sacrum 11.
(2) The sacrum pressing tool XH includes the first member XHa fixed to the inner side of the rear portion of the spats 3 and constituting a part of the base 21H, a second member XHb constituting another part of the base 21H and the pair of protruding portions 22H, and a third member XHc detachably connected between the first member XHa and the second member XHb constituting a part of the base 21H.

### [Other Embodiments]

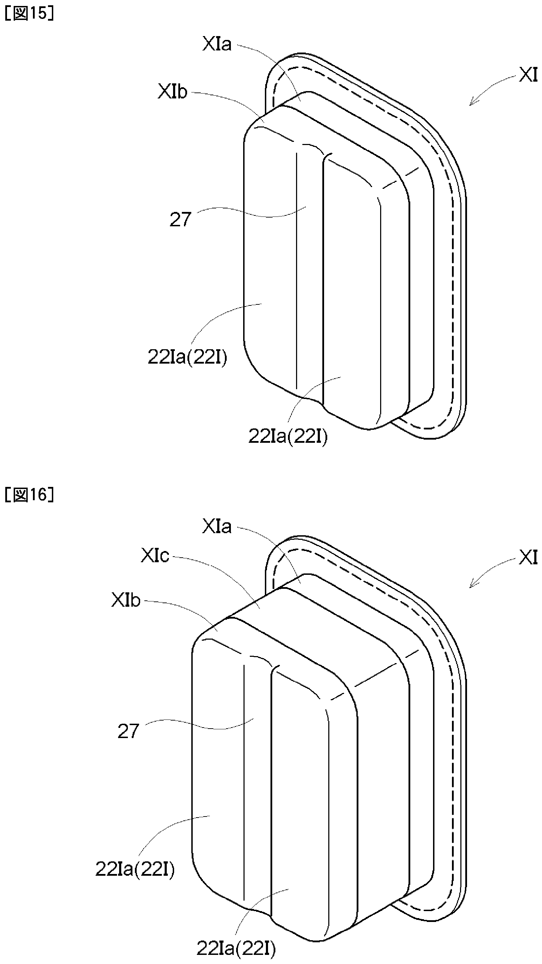

(a) In the sacrum pressing unit Y described above, the third member XHc of the sacrum pressing tool XH may be omitted, or a plurality of such third members XHc may be provided, instead. Further, the first member XHa, the second member XHb and the third member XHc of the sacrum pressing tool XH may be provided as an integrated assembly. Further alternatively, the sacrum pressing tool XH may comprise only the base 21H, with omission of the pair of protruding portions 22H. Moreover, with omission of the fastener belt 4, it may be arranged such that the elasticity of the spats 3 alone causes the sacrum pressing tool XH to move toward the sacrum 11.
(b) The sacrum pressing unit Y described above may employ a modified sacrum pressing tool XI shown in Fig. 15. In the sacrum pressing tool XI used in this embodiment, a first member XIa and a second member XIb are connected to each other, with the height of the second member XIb being set smaller than the height of the second member XHb of the sacrum pressing tool XH described hereinbefore. This arrangement allows reduction in the protruding amount of the rear portion of the spats 3 to the outer side due to the presence of the sacrum pressing tool XI when the sacrum pressing unit Y is worn. Moreover, the circumference of a pair of protruding portions 22I included in the second member XIb are formed in an R-shape and the circumference of a receded portion 27 provided between the pair of protruding portions 221 is also formed in an R-shape. With these R-shape arrangements, pressing faces 22Ia of the pair of protruding portions 22I can press only the sacrum 11 effectively, with no pressing of the iliac bones 12 by these pressing faces 22Ia.
(c) Between the first member XIa and the second member XIb of the sacrum pressing tool HI shown in Fig. 15, a third member XIc shown in Fig. 16 as a "height adjusting member" can be connected. Preferably, the height of this third member XIc is set greater than the height of the second member XIb. With this, the pressing force of the sacrum pressing tool HI to the sacrum 11 can be increased in a reliable manner. Incidentally, the connecting arrangements among the first member XIa, the second member XIb and the third member XIc are same as those of the foregoing embodiments, so detailed explanation thereof will be omitted.

### Industrial Applicability

The present invention can be applied to a sacrum pressing tool and a sacrum pressing unit as a health care appliance for improving motor function, preventing/improving back pain or the like.

### Reference Signs List

- 3:: spats (lower body clothing article)
- 4:: fastener belt
- 10:: coccyx
- 11:: sacrum
- 12:: iliac bones
- 13:: superior posterior iliac spines
- 14:: sacroiliac joints
- 15:: median sacral ridge
- 16:: lumbar vertebra
- 21:: base
- 21B:: base
- 21Bb:: rear face
- 21C:: base
- 21Ca:: outer side face
- 21Cb:: rear face
- 21D:: base
- 21Da:: side face
- 21Db:: rear face
- 21H:: base
- 22:: protruding portion
- 22a:: pressing face
- 22b:: outer side face
- 22c:: receded portion
- 22A:: protruding portion
- 22Aa:: pressing face
- 22Ab:: outer side face
- 22E:: protruding portion
- 22Ea:: pressing face
- 22F:: protruding portion
- 22Fa:: pressing face
- 22H:: protruding portion
- 22Ha:: pressing face
- 22l:: protruding portion
- 22la:: pressing face
- 23:: curved face portion
- 24:: groove portion
- 25:: vertical extension portion
- 25a:: rear face
- 26:: stepped portion
- 31:: pocket
- 32a:: fixing portion
- B:: belt
- B1:: first belt
- B2:: second belt
- F:: direction from base toward sacrum
- H1:: height from top face of protruding portion at stepped portion
- L1:: depth of receded portion
- L2:: width of receded portion
- L3:: width between outer side faces of protruding portions
- L4:: width between a pair of superior posterior iliac spines
- X:: sacrum pressing tool
- XA-XG:: sacrum pressing tools
- XH, XI:: sacrum pressing tool
- XHa, XIa:: first member
- XHb, XIb:: second member
- XHc, XIc:: third member
- Y:: sacrum pressing unit

## Claims

1. A sacrum pressing tool comprising:
a solid pillar-like base; and
a pair of protruding portions protruding in left-right symmetry from opposed side portions of the base;
wherein a width between outer side faces of the pair of protruding portions being set smaller than a width between a pair of the superior posterior iliac spines, so that pressing faces of the pair of protruding portions press a sacrum solely.

2. The sacrum pressing tool of claim 1, wherein the pair of protruding portions are configured such that the respective outer side faces thereof comprise inclined faces which extend closer to each other as they extend away from the base.

3. The sacrum pressing tool of claim 1 or 2, wherein:
the sacrum pressing tool further comprises a belt configured to be fixed to a rear face of the base present opposite to the protruding portions and configured also to be wound around a human body for exerting a pressing force toward the protruding portions; and
the rear face of the base is formed as a convexly curved face.

4. The sacrum pressing tool of claim 1 or 2, wherein:
the sacrum pressing tool further comprises a belt configured to be fixed to a rear face of the base opposite to the protruding portions and configured also to be wound around a human body for exerting a pressing force toward the protruding portions; and
a pair of outer side faces located on the opposed sides of the base are formed as tapered faces that extend closer to each other as they extend away from the protruding portions.

5. The sacrum pressing tool of any one of claims 1-4, wherein the pressing faces are formed as flat faces.

6. The sacrum pressing tool of any one of claims 1-5, further comprising a vertical extension portion that extends vertically from a top face of the base.

7. A sacrum pressing unit comprising:
a sacrum pressing tool having a pressing face for pressing the sacrum, a width between outermost lines of the pressing face being set smaller than a width between a pair of the superior posterior iliac spines; and
a lower body clothing article to which the sacrum pressing tool is fixed to an inner side of a rear portion of the article;
wherein a fixing position of the sacrum pressing tool relative to the lower body clothing article being set so as to allow the pressing face to press the sacrum solely.

8. The sacrum pressing unit of claim 7, wherein:
the sacrum pressing unit further comprises a fastener belt that can be wound around an outer circumferential face of the lower body clothing article; and
in a rear face of the lower body clothing article at least at a position thereof where the sacrum pressing tool is to be located, there is provided a fixing portion for fixing a winding position of the fastener belt.

9. The sacrum pressing unit of claim 7 or 8, wherein the sacrum pressing tool further includes a first member fixed to the rear inner side of the lower body clothing article, a second member having the pressing face and a third member detachably connected between the first member and the second member.
